(19) Europäisches Patentamt
European Patent Office
Office européen des brevets

(11) **EP 4 307 308 A1**

(12) **EUROPEAN PATENT APPLICATION**
published in accordance with Art. 153(4) EPC

(43) Date of publication:
**17.01.2024 Bulletin 2024/03**

(21) Application number: **22767355.5**

(22) Date of filing: **17.02.2022**

(51) International Patent Classification (IPC):
*G16C 20/10* (2019.01)    *G16C 20/30* (2019.01)
*G16C 20/20* (2019.01)    *G16C 20/70* (2019.01)
*G06N 3/08* (2023.01)    *G06N 20/00* (2019.01)

(52) Cooperative Patent Classification (CPC):
**G06N 3/08; G06N 20/00; G16C 20/10; G16C 20/20; G16C 20/30; G16C 20/70**

(86) International application number:
**PCT/KR2022/002340**

(87) International publication number:
**WO 2022/191459 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.03.2021 KR 20210030344**

(71) Applicant: **BNJ Biopharma Inc.**
**Incheon 21983 (KR)**

(72) Inventors:
• **DONG, Jae June**
  **Seoul 03165 (KR)**
• **LEE, Hyeon Geun**
  **Seoul 03737 (KR)**
• **PARK, Tae Hwan**
  **Seoul 07539 (KR)**
• **SHIN, Dong Il**
  **Seongnam-si Gyeonggi-do 13600 (KR)**
• **BAIG, Mohammad Hassan**
  **Seoul 04788 (KR)**

(74) Representative: **IK-IP LTD**
**3 Lloyd's Avenue**
**London, Greater London EC3N 3DS (GB)**

(54) **DRUG DESIGN METHOD AND DEVICE USING SAME**

(57)    The present invention provides a drug design method implemented by a processor and a communication unit, and a device using the same, the drug design method comprising the steps of: receiving the structure of a biological target by means of the communication unit; performing, by means of the processor, reinforcement learning by using the binding affinity with the biological target, with respect to a compound producing model which has been pre-trained to output the molecular structure of a similar compound by inputting the molecular structure of the compound, so as to obtain a reinforcement-learned compound producing model; producing a candidate compound for the biological target by means of the reinforcement-learned compound producing model; and providing the candidate compound.

FIG. 1B

EP 4 307 308 A1

**Description**

[Technical Field]

The present disclosure relates to a drug design method and a device using the same, and more particularly, to a drug design method based on a structural characteristic of a drug and a device for designing a drug using the same.

[Background Art]

With regard to the drug design, studies on drug action pathways have been consistently conducted. Numerous reports and follow-up studies from phenotypic screening to post-selling side-effect monitoring have been conducted. However, the design on the drug action focuses on the studies about an intended therapeutic effect of the drug or frequently occurring side effects so that there are many difficulties in decryption of a mechanism of action for unintended drug effects.

At this time, in the drug design, such as understanding of the drug action, discovering of new drug effects, or predicting of side effects, a research based on an unbiased analysis method for drug action is needed.

In the meantime, it has been reported that drug databases including literature or data including pharmacogenetics, drug-induced gene expression profiles, and drug side effect information are growing rapidly. Simultaneously, the need for drug design using these drug databases is emerging, and drug design methods that enhance the accessibility and usability of drug databases are in the spotlight. At this time, the design method based on the drug database is very excellent in terms of time and cost efficiency and may be highly reproducible by means of the systematic implementation.

As described above, the database-based drug design may be applied to the discovering of candidate materials, but an appropriate method has not been proposed so far.

Therefore, there is a continuous need for a new drug design method based on a drug database.

The background of the present disclosure is described for easier understanding of the present disclosure. It should not be understood to admit the matters described in the background of the present disclosure as a prior art.

[Disclosure]

[Technical Problem]

With regard to the drug design, there have been attempts to predict a drug target, a chemical structure, and a drug action of side effect based on a chemical property of the drug. However, the drug design is useful in the new drug design, but tends to rely on the intrinsic mechanism of the drug. The drug design as described above may cause a problem according to an interaction with a target (for example, virus proteins) which is underrepresented in actual clinical stage.

With more regard to the drug design, there are attempts to randomly create molecules of a candidate material using a neural network based generative model and discover the candidate material therefrom. However, there may be a problem in that the time and the cost required to discover a candidate for a target whose structure is not well known, that is, in the initial drug candidate material stage, are significant.

In the meantime, in order to solve the problems of the drug design system of the related art, the inventors of the present disclosure attempted to evaluate the adequacy of the candidate drug by considering a binding affinity with the target.

With regard to this, the inventors of the present disclosure attempted to select a material which gets a maximum reward according to the binding energy of the target-candidate material in a docking simulation environment using reinforcement learning.

As a result, the inventors of the present disclosure have designed a new drug design system configured to evaluate a structural adequacy of the drug.

To be more specific, the inventors of the present disclosure designed a reward function based on a binding energy of a target-candidate material, and therefrom, designed an agent network which generates a molecule predicted to interact with the biological target.

Specifically, the inventors of the present disclosure attempted to train a reinforcement-learned compound producing model to maximize the increasing binding affinity when unit atoms (for example, C, H, and O) which make up the compound were increased, rather than increasing the affinity by simply increasing the number of molecules which make up the compound.

As a result, the reinforcement-learned compound producing model which was trained to maximize an increasing level of the binding affinity in accordance with the increase of the unit atoms may be constructed.

The reinforcement-learned compound producing model may solve a tendency of the mode collapse which may be shown in a simple binding model of a producing model and a reinforcement learning model trained to increase a total binding energy by simply increasing the number of molecules which make up the compound.

Further, the inventors of the present disclosure attempted to design a reinforcement-learned producing model to produce the compound having a common binding force for two or more biological targets (for example, a plurality of target proteins) according to a modification of the reward function. Further, the inventors of the present disclosure recognized to acquire a compound selectively having a high binding affinity with a single target, and a low binding affinity with another target, in accordance with the modification of the reward function. Therefore, the inventors of the present disclosure were able to expect a therapeutic effect for a plurality of diseases, or selective therapeutic effect with a single drug by providing a candidate drug having an inhibitory activity to a plurality of targets or a target selective inhibitory activity.

Moreover, the inventors of the present disclosure were able to expect to provide a candidate material similar to an existing drug with respect to a new biological target (or a ligand thereof) which was not trained to the artificial intelligence by a reinforcement learning model based new drug design system in the related art, that is, which does not have data.

Specifically, the inventors of the present disclosure were able to expect that a new drug design system could contribute to study to improve a global pandemic situation, such as coronavirus disease 19 (COVID-19).

Moreover, the inventors of the present disclosure were able to evaluate adequacy between drugs by considering all action mechanism and structural characteristic for a candidate drug and a target to provide a more reliable design result for drugs than the drug design system of the related art.

Accordingly, an object to be achieved by the present disclosure is to provide a drug design method and a device configured to produce a candidate drug using a reward according to a binding affinity based on the reinforcement learning model.

Objects of the present disclosure are not limited to the above-mentioned objects, and other objects, which are not mentioned above, may be clearly understood by those skilled in the art from the following descriptions.

[Technical Solution]

In order to achieve the above-described objects, A drug design method according to an exemplary embodiment of the present disclosure is provided. The method is a drug design method implemented by a processor and a communication unit including the steps of: receiving a structure of a biological target, by means of the communication unit; performing, by means of the processor, reinforcement learning by using the binding affinity with the biological target, with respect to a compound producing model which has been pre-trained to output a molecular structure of a similar compound by inputting the molecular structure of the compound so as to obtain a reinforcement-learned compound producing model; producing a candidate compound for the biological target by means of the reinforcement-learned compound producing model; and providing a candidate compound.

According to a feature of the present disclosure, the step of producing a candidate compound may include the steps of: producing a plurality of candidate compounds using the reinforcement-learned compound producing model; determining a binding affinity for the biological target and each of the plurality of candidate compounds; and determining a candidate compound for the biological target, among the plurality of candidate compounds, based on the binding affinity for each thereof.

According to another feature of the present disclosure, the step of determining a binding affinity may include the step of: determining a reward score for each thereof, using a reward function configured to evaluate a binding energy with a biological target, in a docking simulation environment, and the step of determining a candidate compound may include the step of: determining a candidate compound for the biological target, among the plurality of candidate compounds, based on the reward score.

According to still another feature of the present disclosure, the step of determining a candidate compound may include the step of determining a compound with a maximum reward score, among the plurality of candidate compounds, as a candidate compound for a biological target.

According to still another feature of the present disclosure, the reward function may be configured to evaluate the binding affinity based on binding energy distribution of the previously received standard biological target and a standard compound.

According to still another feature of the present disclosure, the reward function may be configured to determine the binding affinity score with a value between 0 and 1.

According to still another feature of the present disclosure, the reward function may be configured to get a high reward score as the binding affinity with the biological target is lower. Further, the step of determining a candidate compound may further include the step of: determining a candidate compound with a low binding affinity with the biological target, among the plurality of candidate compounds, based on the reward score.

According to still another feature of the present disclosure, the drug design method may further include the steps of: before the step of performing the reinforcement learning, receiving a molecular structure of a compound for training; converting the molecular structure of the compound for training into a simplified molecular-input line-entry system (SMILES) code for training; and training the compound producing model to output a similar SMILES code to the compound for training by inputting the SMILES code for training.

According to still another feature of the present disclosure, the step of producing a candidate compound may include the steps of: producing a plurality of SMILES codes using the reinforcement-learned compound producing model; determining a plurality of candidate compounds based on the plurality of SMILES codes; determining a binding affinity for the biological target and each of the plurality of candidate compounds; and determining a candidate compound for the biological target, among the plurality of candidate compounds, based on the binding affinity for each thereof.

According to still another feature of the present disclosure, the reinforced compound producing model may include an agent network. Further, the step of performing reinforcement learning may include the step of: updating the agent network using a reward function configured to evaluate the binding affinity based on the binding energy with the biological target.

According to still another feature of the present disclosure, the biological target may be at least one of a specific region of virus, a virus protein, such as main protease (Mpro) or polymerase associated with the activity of coronavirus and severe acute respiratory syndrome coronavirus.

In order to achieve the above-described objects, a drug design device according to an exemplary embodiment of the present disclosure is provided. The device includes a communication unit configured to receive a structure for a biological target; and a processor configured to communicate with the communication unit. At this time, the processor is configured to perform reinforcement learning by using the binding affinity with the biological target, with respect to a compound producing model which has been pre-trained to output a molecular structure of a similar compound by inputting the molecular structure of the compound so as to obtain a reinforcement-learned compound producing model, produce a candidate compound for the biological target by means of the reinforcement-learned compound producing model; and provide the candidate compound.

According to a feature of the present disclosure, the processor may be further configured to produce a plurality of candidate compounds using the reinforcement-learned compound producing model; determine a binding affinity for the biological target and each of the plurality of candidate compounds; and determine a candidate compound for the biological target, among the plurality of candidate compound, based on the binding affinity for each thereof.

According to another feature of the present disclosure, the processor may be further configured to determine a reward score for each thereof, using a reward function configured to evaluate a binding energy with a biological target, in a docking simulation environment, and determine a candidate compound for the biological target, among the plurality of candidate compounds, based on the reward score.

According to still another feature of the present disclosure, the processor may be further configured to determine a compound with a maximum reward score, among the plurality of candidate compounds, as a candidate compound for a biological target.

According to still another feature of the present disclosure, the reward function may be configured to evaluate the binding affinity based on binding energy distribution of the previously received standard biological target and a standard compound.

According to still another feature of the present disclosure, the reward function may be configured to determine the binding affinity score with a value between 0 and 1.

According to still another feature of the present disclosure, the reward function may be configured to get a high reward score as the binding affinity with the biological target is lower. At this time, the processor may be further configured to determine a candidate compound with a low binding affinity with the biological target, among the plurality of candidate compounds, based on the reward score.

According to still another feature of the present disclosure, the communication unit is further configured to receive a molecular structure of a compound for training, and the processor may be further configured to convert the molecular structure of the compound for training into a simplified molecular-input line-entry system (SMILES) code and train the compound producing model to output a similar SMILES code to the compound for training by inputting the SMILES code for training.

According to still another feature of the present disclosure, the processor may be further configured to produce a plurality of SMILES codes using the reinforcement-learned compound producing model; determine a plurality of candidate compounds based on the plurality of SMILES codes, determine a binding affinity for the biological target and each of the plurality of candidate compounds, and determine a candidate compound for the biological target, among the plurality of candidate compound, based on the binding affinity for each thereof.

According to still another feature of the present disclosure, the reinforced compound producing model may include an agent network. At this time, the processor may be further configured to update the agent network using a reward function configured to evaluate the binding affinity based on the binding energy with the biological target.

According to still another feature of the present disclosure, the biological target may be at least one of a specific region of virus, a virus protein, such as main protease (Mpro) or polymerase associated with the proliferation of coronavirus and severe acute respiratory syndrome coronavirus.

Hereinafter, the present disclosure will be described in detail with reference to the examples. However, the exemplary embodiments are only for illustrative purposes of the present disclosure, and therefore the scope of the present disclosure should not be construed as being limited by these exemplary embodiments.

[Advantageous Effects]

According to the present disclosure, a neural network based generative model is used to randomly produce a molecule of a candidate material to overcome a limitation of a drug design system of the related based on the production of the candidate material therefrom.

To be more specific, according to the present disclosure, a drug design system of the related art which consumes significant time and cost in a step of discovering a candidate material for a target whose structure is not well known, that is, at the step of discovering an initial medicine candidate material.

Specifically, according to the present disclosure, a new drug design system configured to select a material which gets a maximum reward according to the binding energy of the target-candidate material in a docking simulation environment using reinforcement learning model may be provided.

Accordingly, according to the present disclosure, a new material similar to an existing drug, with respect to a new biological target which is not trained to the artificial intelligence of the related art by a reinforcement learning model based new drug design system, that is, which does not have data, may be provided.

Therefore, according to the present disclosure, a highly reliable design result with a less time and cost compared with the drug design system of the related art may be provided.

Specifically, according to the present disclosure, a plurality of new and effective compounds having a high binding affinity with a biological target may be provided to contribute to the efficiency increase of new drug development (de novo drug design).

Moreover, the present disclosure may contribute to the study to improve the global pandemic situation.

The effects according to the present disclosure are not limited to the contents exemplified above, and more various effects are included in the present disclosure.

[Description of Drawings]

FIG. 1A is a schematic diagram for explaining a drug design system according to an exemplary embodiment of the present disclosure.

FIG. 1B is a schematic diagram of a configuration of a drug design device according to an exemplary embodiment of the present disclosure.

FIG. 1C is a schematic diagram for explaining a user mobile device which is provided with information from a drug design device according to an exemplary embodiment of the present disclosure.

FIG. 2A is a schematic flowchart for explaining a drug design method according to an exemplary embodiment of the present disclosure.

FIG. 2B illustrates a procedure of reinforcement learning according to an exemplary embodiment of the present disclosure.

FIG. 2C illustrates active data of a ChEMBL database for designing a reward function which is applied to a reinforcement-learned compound producing model used for various exemplary embodiments of the present disclosure.

FIG. 2D illustrates a procedure of determining a candidate compound by a drug design procedure according to an exemplary embodiment of the present disclosure.

FIG. 3 illustrates a pre-training procedure of a compound producing model used for various exemplary embodiments of the present disclosure.

FIG. 4 illustrates a structure of compound producing model used for various exemplary embodiments of the present disclosure.

FIGS. 5A to 5D illustrate an evaluation result of a reinforcement-learned compound producing model used for various exemplary embodiments of the present disclosure.

FIGS. 6A and 6B illustrate comparison of characteristics of candidate compounds produced by each of a reinforcement-learned compound producing model used for various exemplary embodiment of the present disclosure and the previously trained compound producing model.

[Modes of the Invention]

Advantages and characteristics of the present disclosure and a method of achieving the advantages and characteristics will be clear by referring to exemplary embodiments described below in detail together with the accompanying drawings. However, the present disclosure is not limited to the following exemplary embodiments but may be implemented in various different forms. The exemplary embodiments are provided only to complete the disclosure of the present disclosure and to fully provide a person having ordinary skill in the art to which the present disclosure pertains with the category of the disclosure, and the present disclosure will be defined by the appended claims.

Although the terms "first", "second", and the like are used for describing various components, these components are not confined by these terms. These terms are merely used for distinguishing one component from the other components. Therefore, a first component to be mentioned below may be a second component in a technical concept of the present disclosure.

Like reference numerals generally denote like elements throughout the specification.

The features of various embodiments of the present disclosure may be partially or entirely bonded to or combined with each other and may be interlocked and operated in technically various ways understood by those skilled in the art, and the embodiments may be carried out independently of or in association with each other.

For clarity of interpretation of the present specification, terms used in the present specification will be defined below.

The term "biological target" used in the present disclosure may refer to an object which causes a disease or some symptoms so as to be suppressed from being activated or produced (or synthesized).

Desirably, the biological target may be a virus itself or a virus protein, but is not limited thereto. For example, the biological target may be a main protease (Mpro) or RNA-dependent RNA polymerase (RdRp) associated with proliferation of coronavirus or severe acute respiratory syndrome coronavirus.

According to the characteristic of the present disclosure, the biological target may be a material whose structure (specifically, a protein structure) is known.

The term "candidate compound" used in the present specification may refer to a compound which is bound to the biological target to inhibit the activity or production of the biological target to block the pathogenesis.

For example, the candidate compound is bound to a core portion (specifically, protease or polymerase) associated with proliferation or activity of the virus to inhibit the activity thereof or block a virus infiltration path.

In the present specification, the candidate compound may be exchangeably used with a candidate drug or a candidate material.

Moreover, the candidate compound may also be interpreted as the same meaning as ligand.

The term "binding affinity" used in the present specification may refer to a binding degree of a compound and a biological target.

In the meantime, the binding affinity may correspond to a binding energy, but is not limited thereto.

For example, a compound having a high binding affinity with the biological target may refer to a compound which may be bound with a biological target with a high binding energy in a docking simulation environment. Moreover, this may inhibit the activity of the biological target so that it may mean that it is likely to be a candidate drug which may also inhibit the activity of the target virus.

The term "compound producing model" used in the present specification may refer to a model which has been previously trained to output a molecular structure of a similar compound by inputting a molecular structure of the compound.

That is, the compound producing model in the present specification may be interpreted to be the same as the previously trained compound producing model.

At this time, a molecular structure of the compound which is input to the compound producing model may be a simplified molecular-input line-entry system (SMILES) code type, but is not limited thereto.

For example, the compound producing model may be trained to output a similar SMILES code by inputting a SMILES code for the biological active molecules acquired from a ChEMBL database which provides information about biological active molecules with a plurality of drug-like characteristics.

That is, the compound producing model may be a machine learning algorithm based generative model which learns a distribution and a characteristic of the existing compound to produce a molecular structure (or SMILES code) of a new compound.

At this time, the compound producing model may be at least one of a long short term memory network (LSTM) model, a recurrent neural network (RNN) model, a convolution neural network (CNN) model, a gated recurrent unit (GRU) model, and a transformer model.

According to a characteristic of the present disclosure, the compound producing model may be an LSTM model, but is not limited thereto.

The term "reinforcement-learned compound producing model" used in the present disclosure may refer to a model having an agent trained to maximize the reward according to the binding affinity with the biological target.

To be more specific, the reinforcement learned-compound producing model may be a model learned to produce a compound with a high adequacy predicted to interoperate with the biological target, based on a reward function based on a binding energy of the biological target-candidate compound.

That is, the reinforcement-learned compound producing model may be a model which is reinforcement-learned to produce a compound with a high binding affinity according to a structure of a biological target, by the reward function, unlike the compound producing model (or a previously trained compound producing model) trained to randomly produce the compound.

At this time, a reinforcement-learned compound producing model may be a model trained to maximize the increasing bonding affinity when unit atoms (for example, C, H, and O) which make up the compound are increased, rather than increasing the affinity by simply increasing the number of molecules which make up the compound.

That is, the reinforcement-learned compound producing model may be a model trained to maximize an increasing level of the binding affinity in accordance with the increase of the unit atoms.

The reinforcement-learned compound producing model may solve a tendency of the mode collapse which may be generated in a simple binding model of a producing model and a reinforcement learning model trained to increase a total binding energy by simply increasing the number of molecules which make up the compound.

Further, the reinforcement-learned compound producing model may be trained to increase a common binding affinity for two or more biological targets (for example, a plurality of target proteins). That is, the reinforcement-learned compound producing model may provide a candidate drug having an inhibitory activity against a plurality of targets to provide therapeutic effects for a plurality of diseases.

Moreover, the reinforcement-learned compound producing model may be trained to increase a binding affinity with one biological target while selectively lowering a binding affinity with another biological target.

Such a biological target may be set in various manners depending on a design of the learning reward function. Here, the "reward function" may refer to a function designed to evaluate a binding energy of the biological target and the compound.

To be more specific, the reward function may mean the functionalization a binding affinity of the biological target and the compound in a docking simulation environment.

For example, the binding affinity B(A) of the biological target and the compound may be calculated by the following Equation 1.

[Equation 1]

$$B(A) = \max (\text{binding energy/empirical minimum binding energy of compound}, 0)$$

At this time, the binding affinity may have a value between 0 and 1. With regard to this, Equation 1 may be an equation designed to calculate a binding affinity based on a distribution of activity data of a biological target and a ligand (compound), acquired from a database of a known bio active molecules. However, it is not limited thereto, and the binding affinity may be determined by more various methods.

That is, the term "standard biological target" used in the present specification may be the known biological active molecules having a drug-like characteristic which may be acquired from a database, such as ChEMBL. And "binding energy of standard compound" may mean active energy of a biological active molecule which may be acquired from a database, such as ChEMBL.

In the meantime, the reward of the compound producing model may be determined based on Equation 2.

[Equation 2]

$$QED*0.1 + [\text{Docking free energy}]*0.9$$

Here, a quantitative estimate of drug-likeness (QED) is a quantitative estimate value of the drug likeness and may be a parameter which may change the weight.

That is, the reinforcement-learned component producing model to receive a maximum reward according to the binding affinity, with a trained agent, may produce a candidate compound more appropriate to the biological target than the previous compound producing model.

In the meantime, it is not limited thereto, and various weights (for example, - weight) may be applied to a QED value of a reward function so that the compound producing model may exclude a compound with a low affinity, or selectively also produce a compound which has a low binding affinity with one biological target but has a high binding affinity with another biological target. Moreover, the compound producing model may also produce a compound having a high binding affinity with a plurality of biological targets.

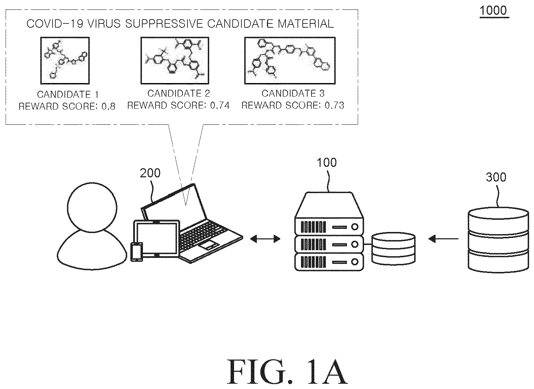

Hereinafter, a drug design device according to various exemplary embodiments of the present disclosure will be described in detail with reference to FIGS. 1A to 1C.

First, referring to FIG. 1A, a drug design system 1000 may be a system configured to provide information about a candidate drug having a suppressive ability against a biological target. At this time, the drug design system 1000, based on a binding affinity of the biological target and a compound, may be configured by a drug design device 100 configured to determine a candidate compound, a user mobile device 200, and an information providing server 300 which provides various information about the biological target and the compound.

First, the drug design device 100 may include a general-purpose computer, a laptop, and/or a data server which perform various computations to evaluate a drug based on data about a biological target or a compound provided from the information providing server 300. At this time, the user mobile device 200 may be a device which accesses a web server which provides a web page for a candidate drug or a mobile web server which provides a mobile web site, but is not limited thereto.

Specifically, the drug design device 100 may be configured to receive data about a structure of a biological target provided from the information providing server 300 and determine a binding affinity of the received biological target and the compound using an artificial intelligence algorithm based compound producing model to provide a compound having a high binding affinity as a candidate compound.

The drug design device 100 may provide data obtained by analyzing a candidate drug for the biological target to the user mobile device 200.

Data provided from the drug design device 100 may be provided to a web page through a web browser installed in the user mobile device 200 or provided as an application or a program type. In various exemplary embodiments, the data may be provided to be included in a platform in a client-server environment.

Next, the user mobile device 200 is an electronic device which provides a user interface to request a drug design for the biological target and represent analysis result data, and may include at least one of a smart phone, a tablet personal computer (PC), a laptop, and/or a PC.

The user mobile device 200 receives a candidate drug analysis result for the biological target from the drug design device 100 and may display the received result through a display unit. Here, the analysis result may include a structure of the candidate compound with a high binding affinity with the biological target, a SMILES code thereof, and a binding affinity (or, a reward score). At this time, a plurality of candidate compounds may be provided.

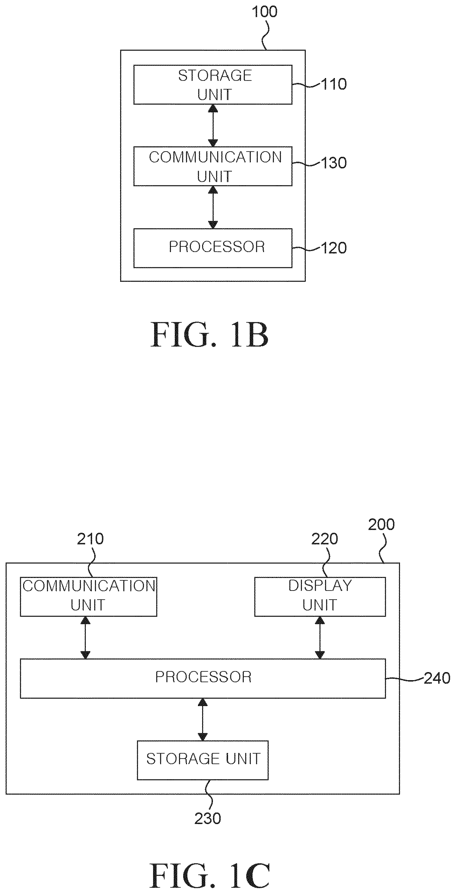

Next, referring to FIG. 1B, a component of the drug design device 100 of the present disclosure will be described in detail.

Referring to FIG. 1B, the drug design device 100 includes a storage unit 110, a communication unit 120, and a processor 130.

First, the storage unit 110 may store various data for evaluating a candidate drug for a biological target. According to various embodiments, the storage unit 110 may include at least one type of storing media of flash memory type, hard disk type, multimedia card micro type, card type memory (for example, SD or XD memory), RAM, SRAM, ROM, EEPROM, PROM, magnetic memory, magnetic disk, and optical disk.

The communication unit 120 communicatively connects the drug design device 100 with the external device. The communication unit 120 is connected to the user mobile device 200, and furthermore to the information providing server 300 using wired/wireless communication to transmit and receive various data. Specifically, the communication unit 120 may receive a structure of the biological target, and furthermore data about the known compound, such as ChEMBL from the information providing server 300. Further, the communication unit 120 may transmit an analysis result to the user mobile device 200.

The processor 130 is operatively connected to the storage unit 110 and the communication unit 120, and may perform various instructions to analyze a candidate drug for the biological target.

Specifically, the processor 130 receives a structure for the biological target from the information providing server 300 through the communication unit 120 and may provide a compound having a high binding energy with the biological target in the docking simulation environment as a candidate compound.

At this time, the processor 130 may be based on the reinforcement-learned compound producing model to produce a compound with a high adequacy predicted to interoperate with the biological target, based on a reward function based on a binding energy of the biological target-candidate compound.

Accordingly, the user may be provided with a new compound which suppresses the activity of a biological target whose structure is only known or suppresses replication thereof, by means of the user mobile device 200.

That is, the user may reduce a time and a cost spent in the initial drug candidate discovering stage.

As described above, according to the present disclosure, a plurality of new (novelty) and effective (validity) compounds having a high binding affinity with a biological target may be provided to contribute to the efficiency increase of new drug development (de novo drug design).

In the meantime, referring to FIG. 1C, the user mobile device 200 includes a communication unit 210, a display unit 220, a storage unit 230, and a processor 240. The communication unit 210 connects the user mobile device 200 to communicate with the external device. The communication unit 210 is connected to the drug design device 100 using wired/wireless communication to transmit and receive various data. Specifically, the communication unit 210 may receive an analysis result associated with the biological target from the drug design device 100.

The display unit 220 may display various interface screens to represent an analysis result associated with the biological target.

According to various embodiments, the display unit 220 may include a touch screen, and for example, may receive touch, gesture, proximity, drag, swipe, or hovering input which uses an electronic pen or a part of a body of the user.

The storage unit 230 may store various data used to provide a user interface to represent result data. According to various embodiments, the storage unit 230 may include at least one type of storage medium of flash memory type, hard disk type, multimedia card micro type, card type memory (for example, SD or XD memory), random access memory (RAM), static random access memory (SRAM), read only memory (ROM), electrically erasable programmable read only memory (EEPROM), programmable read only memory (PROM), magnetic memory, magnetic disk, and optical disk.

The processor 240 is operatively connected to the communication unit 210, the display unit 220, and the storage unit 230, and may perform various instructions to provide a user interface to represent result data.

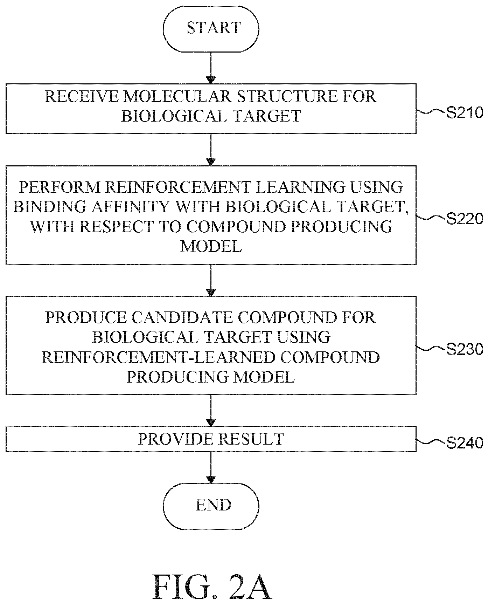

Hereinafter, a candidate compound producing method using a reinforcement-learned compound producing model which is used for various exemplary embodiments of the present disclosure will be described with reference to FIGS. 2A to 2D.

First, referring to FIG. 2A, a molecular structure for the biological target is received (S210). Next, reinforcement learning is conducted on the compound producing model (S220), and a candidate compound for the biological target is produced by the reinforcement-learned model (S230). Finally, a result is provided (S240).

To be more specific, in the step of receiving a molecular structure for the biological target (S210), a protein structure for a biological target, such as virus protein (for example, Mpro) is received.

Next, in the step of performing the reinforcement learning (S220), for a compound producing model which has been pre-trained to output the molecular structure of a similar compound by inputting the molecular structure of the compound, reinforcement learning is performed using the binding affinity with the biological target.

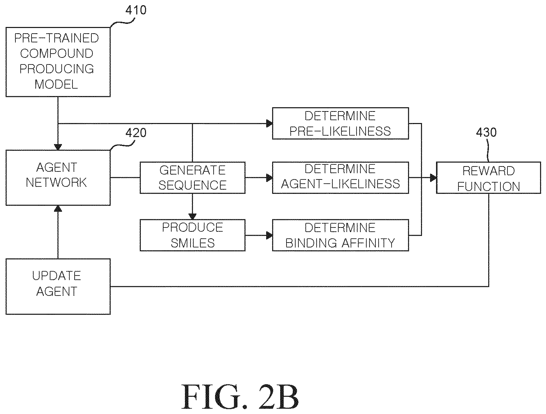

For example, referring to FIG. 2B together, in the step of performing the reinforcement learning (S220), a prior likelihood is determined from a prior network of the pre-trained compound producing model 410, and a sequence is produced from an agent network 420, and then an agent likelihood is determined. Simultaneously, the SMILES code is produced and a binding affinity with a previously received biological target is determined. Next, an agent is trained to produce a compound to maximize a reward, by a reword function 430 which is designed to evaluate a binding energy of the biological target and the compound, and the trained content is updated to produce a reinforcement-learned compound producing model.

At this time, the reward function may be an equation designed to calculate a binding affinity based on a distribution of activity data of a biological target and a ligand (compound), acquired from a database of a known bio active molecules.

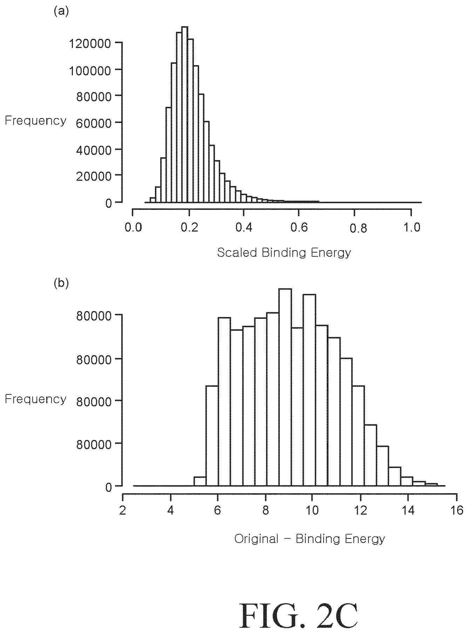

For example, referring to FIG. 2C (a), a scaled binding energy (or prior reward score) from the activity data of the ChEMBL database rarely has values higher than 0.5, but is generally distributed at low values. Further, referring to FIG. 2C (b), a binding energy before normalization has a minimum value of -15.21 kcal/mol and is relatively uniformly distributed.

At this time, the reward function may be configured to be scaled in a value between 0 and 1 by assuming a range of the binding energy as 0 to -15.21 kcal/mol, based on a binding energy distribution acquired from the actual biological target-compound activity experiment data, such as ChEMBL database.

According to the characteristic of the present disclosure, the reward function may be calculated by the following Equation 1.

[Equation 1]

$$B(A) = \max \text{ (binding energy/empirical minimum binding energy of compound, 0)}$$

However, it is not limited thereto.

Referring to FIG. 2A again, as a result of the step of performing the reinforcement learning (S220), a compound producing model having an agent on which the reinforcement learning is conducted, that is trained, may be built. In the meantime, the step of performing the reinforcement learning (S220) is not limited to the above-described procedure. For example, in the step of performing the reinforcement learning (S220), the reinforcement learning may be performed on the compound producing model which outputs the similar SMILES code by inputting the SMILES code representing the molecular structure of the compound. To be more specific, in the step of performing the reinforcement learning (S220), the compound producing model may be trained to achieve a high reward score when a new SMILE code having a high binding affinity with the biological target previously received is produced.

Next, in the step of producing a candidate compound (S230), with respect to a plurality of candidate compounds which is randomly produced by the compound producing model, a binding affinity with the biological target is determined, and a candidate compound for the biological target, among a plurality of candidate compounds, is determined based on the binding affinity.

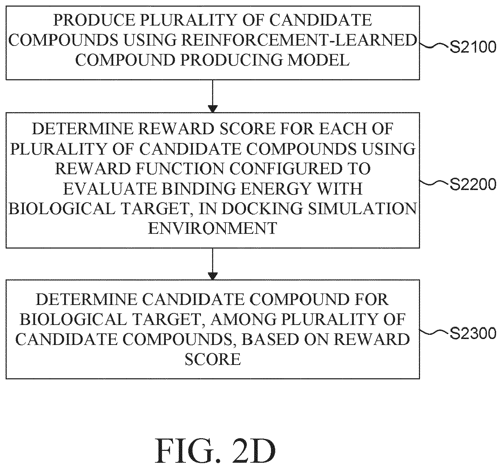

To be more specific, referring to FIG. 2D together, in the step of producing a candidate compound (S230), a plurality of candidate compounds is randomly produced by the compound producing model (S2100). Next, in the docking simulation environment, a reward score for each thereof may be determined by a reward function configured to evaluate a binding energy with the biological target (S2200). Next, a candidate compound for the biological target, among a plurality of candidate compounds, may be determined based on the reward score (S2300).

For example, in the step of determining the candidate compound (S2300), among the plurality of compounds, a compound having a maximum reward score, compounds having a higher reward score, or compounds having a reward score which is equal to or higher than a predetermined level may be determined as candidate compounds (or candidate drugs) for the biological target.

Moreover, when a plurality of biological targets is provided, in the step of determining a candidate compound (S2300), a candidate compound having a high binding affinity with the plurality of biological targets may be determined according to a previously determined reward function. That is, in the step of determining the candidate compound (S2300), a candidate compound which has an inhibitory activity against the plurality of targets and provides a therapeutic effect for the plurality of diseases may also be provided.

In the step of determining a candidate compound (S2300), a candidate compound which selectively has a low binding force with one biological target, but has a high binding force with another biological target may also be determined according to the predetermined reward function.

As described above, in the step of producing the candidate compound (S230), the candidate compound for the biological target may be finally determined based on the binding affinity by the similar method to the reinforcement learning procedure, but is not limited thereto. For example, in the step of randomly producing a plurality of candidate compounds (S2100), the plurality of candidate compounds which is randomly produced from the reinforcement-learned compound producing model may be compounds which have a high binding affinity with the biological target. Therefore, without performing the step of determining a reward score (S2200), a plurality of candidate compounds which is randomly produced in the step of determining a candidate compound (S2300) may be determined as a final candidate compound having a high binding affinity with the biological target.

Referring to FIG. 2A again, in the step of providing a result (S240), compounds with a high binding affinity with the biological target may be provided as candidate compounds.

That is, a plurality of novel and valid compounds having a high binding affinity with the biological target may be provided by the procedures as described above.

Therefore, the present disclosure may contribute to efficiency increase of the de novo drug design. Specifically, according to the present disclosure, a new material similar to an existing drug, with respect to a new biological target which is not trained to the artificial intelligence by a reinforcement learning model based new drug design system, that is, which does not have data may be provided. Therefore, according to the present disclosure, a highly reliable design result with a less time and cost may be provided as compared with the drug design system of the related art.

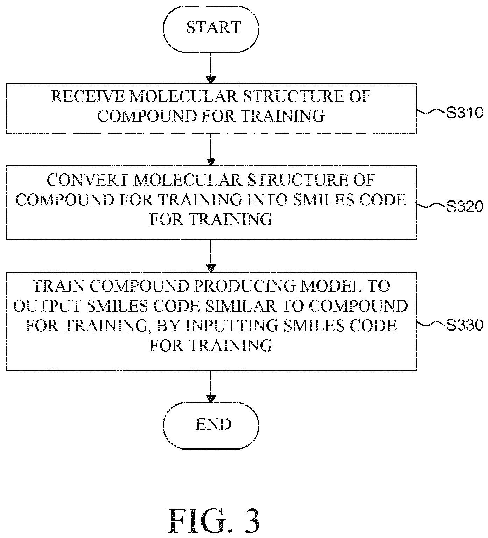

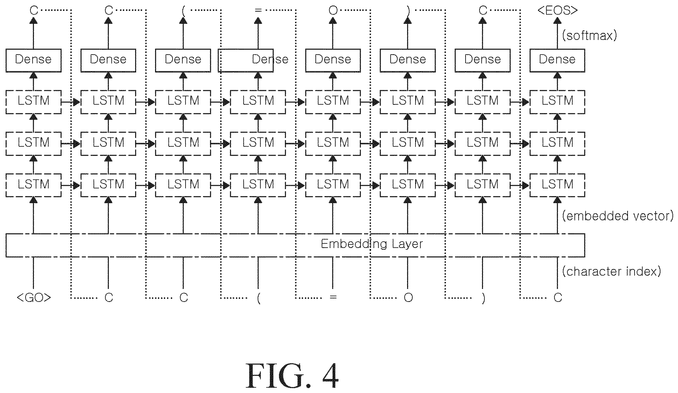

Hereinafter, a learning method of a pre-trained compound producing model applied to various exemplary embodiments of the present disclosure and a structure thereof will be described with reference to FIGS. 3 and 4.

At this time, the compound producing model may be at least one of a long short term memory network (LSTM) model, a recurrent neural network (RNN) model, a convolution neural network (CNN) model, a gated recurrent unit (GRU) model, and a transformer model.

According to a characteristic of the present disclosure, the compound producing model may be an LSTM model, but is not limited thereto.

First, referring to FIG. 3, in order to design a drug design model used for various exemplary embodiments of the present disclosure, a molecular structure of a compound for training is received (S310). Next, the molecular structure of the compound for training is converted into the SMILES code (S320). Next, the artificial intelligence algorithm based compound producing model is trained to output a SMILES code similar to the structure of the compound for training by inputting the SMILES code of the compound for training (S330).

For example, in the step of receiving a molecular structure of the compound (S230), a molecular structure of the compound for training may be received from the ChEMBL database which provides information about the plurality of drug-like biological active molecules. Next, in the step of converting the molecular structure of the compound into the SMILES code (S320), the SMILES code for the existing compound may be produced. Finally, in the learning step (S330), the compound producing model may be trained to output the similar SMILES code by inputting the SMILES code for biological active molecules acquired from the ChEMBL database.

That is, the pre-trained compound producing model which is a machine learning algorithm based generative model which learns a distribution and a characteristic of the existing compound to produce a molecular structure (or SMILES code) of a new compound may be built.

At this time, referring to FIG. 4, the pre-trained compound producing model may include an input layer which receives the SMILES code, an embedding layer which generates an embedded vector from the input SMILES code, an encoding layer which is formed by triple layered LSTM, and a decoding layer which outputs a new SMILE code by means of the full connection.

To be more specific, when CC(=O)C which is the SMILES code of acetone is input through the input layer, a new SMILES code may be output through softmax after passing through the embedding layer, the encoding layer, and the decoding layer.

In the meantime, the structure and the learning method of the pre-trained compound producing model are not limited to the above-described structure and method.

Evaluation: Evaluation of reinforcement-learned compound producing model

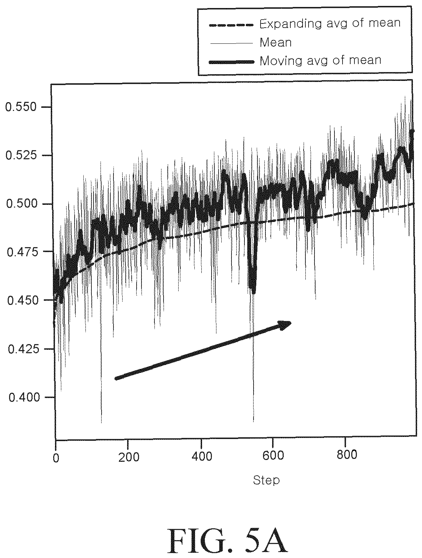

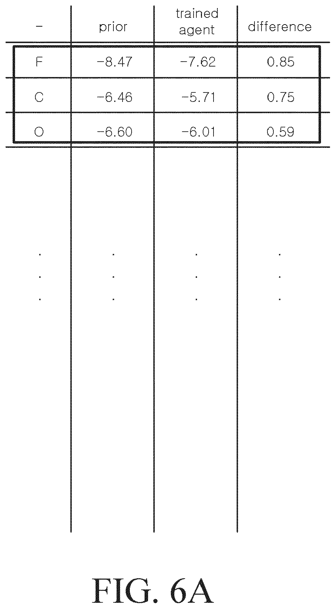

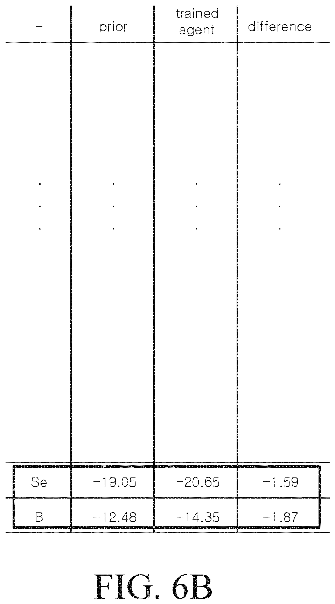

Hereinafter, an evaluation result of the reinforcement-learned compound producing model applied to various exemplary embodiments of the present disclosure will be described with reference to FIGS. 5A to 5D, 6A, and 6B.

At this time, the reinforcement-learned compound producing model may be configured to produce a candidate compound having a high binding affinity with the biological target, such as main protease (Mpro) or RNA dependent RNA polymerase (RdRp) associated with proliferation of coronavirus. However, the biological target is not limited thereto and may also be a site (or a region) whose structure associated with activity or replication of the virus has been identified.

First, referring to FIG. 5A, a learning curve according to the number of repeated steps of reinforcement learning on the compound producing model is illustrated. To be more specific, it appears that the binding energy with coronavirus Mpro improves as reinforcement learning is performed.

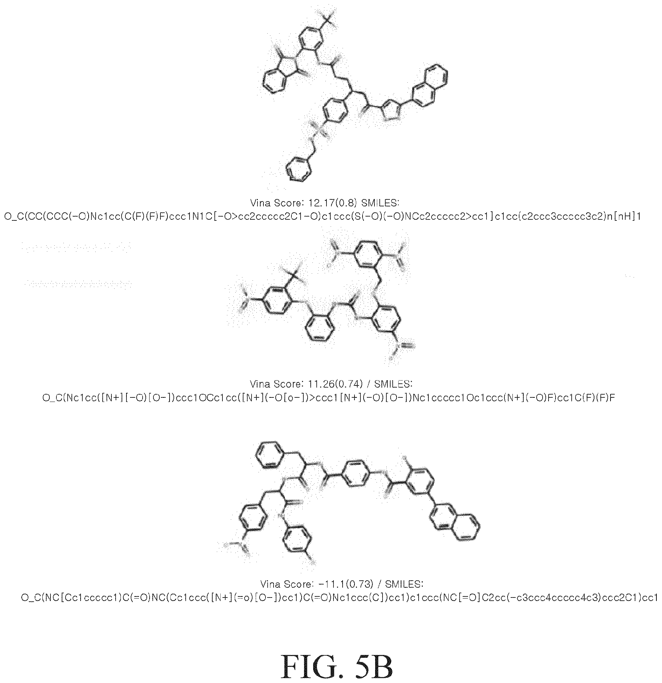

Referring to FIG. 5B (a), (b), and (c) together, three candidate compounds for coronavirus Mpro which are provided by the reinforcement-learned compound producing model have high binding energies of -12.71 kcal/mol, -11.26 kcal/mol, and -11.1 kcal/mol and have high reward scores of 0.8, 0.74, and 0.73, respectively.

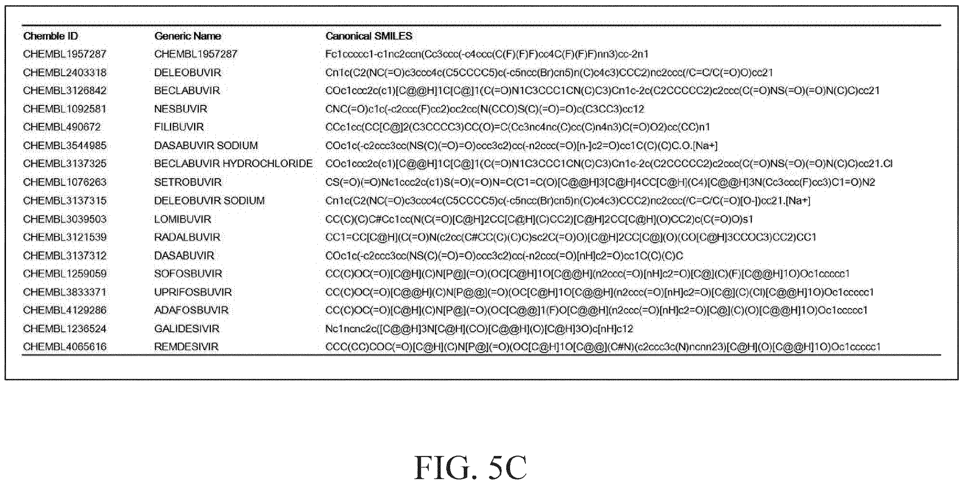

Referring to FIG. 5C together, together with the SMILES code of a compound having a high binding affinity with coronavirus protease which is produced by the reinforcement-learned compound producing model used for various exemplary embodiments of the present disclosure, ChEMBL ID and a component name (generic name) are illustrated. These 46 compounds may have an inhibitory activity against the protease associated with the activity of the coronavirus and the replication thereof so that the compounds may be provided as candidate materials for treatment of the coronavirus infections.

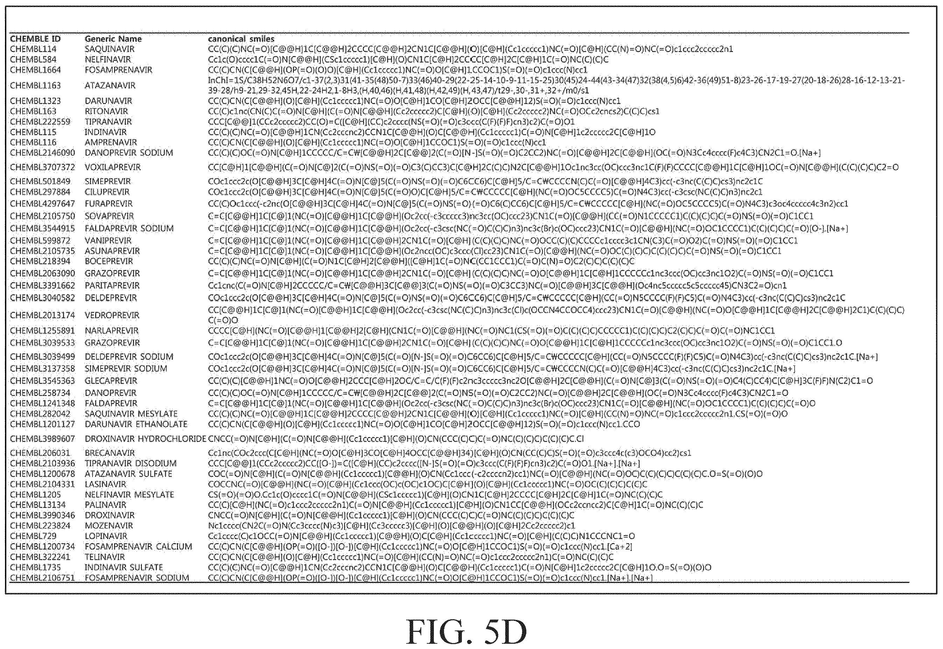

Referring to FIG. 5D, together with the SMILES code of a compound having a high binding affinity with coronavirus RNA-dependent RNA polymerase (RdRp) which is produced by the reinforcement-learned compound producing model used for various exemplary embodiments of the present disclosure, ChEMBL ID and a component name (generic name) are illustrated. These 17 compounds may have an inhibitory activity against the polymerase associated with the activity of the coronavirus so that the compounds may be provided as candidate materials for treatment of the coronavirus infections.

That is, the reinforcement-learned compound producing model used for various exemplary embodiments of the present disclosure may provide a novel compound having a high binding energy associated with the reaction with the biological target as a candidate drug. That is, the candidate drug is bound with the biological target with a high affinity to suppress the activity thereof.

Referring to FIGS. 6A and 6B, characteristics of compounds produced from the reinforcement-learned compound producing model applied to various exemplary embodiments of the present disclosure, that is, a model having a trained agent network and a model before reinforcement-learning (that is, a pre-trained model) having a prior network are compared and illustrated.

To be more specific, referring to FIG. 6A, the reinforcement-learned compound producing model applied to various exemplary embodiments of the present disclosure tends to add fluorine (F), carbon (C), and oxygen (O) to the compound to bind to the coronavirus Mpro, differently from the pre-trained model. Moreover, referring to FIG. 6B, the reinforcement-learned compound producing model applied to various exemplary embodiments of the present disclosure has a low tendency to add metalloid elements, such as boron (B) and selenium (Se), unlike the pre-trained model.

That is, this result may mean that the reinforcement-learned compound producing model applied to various exemplary embodiments of the present disclosure produces a compound with an improved structure more than the pre-trained compound producing model.

Although an exemplary embodiment of the present disclosure has been described in detail with reference to the accompanying drawings, the present disclosure is not limited thereto and may be embodied in many different forms without departing from the technical concept of the present disclosure. Therefore, the exemplary embodiments of the present disclosure are provided for illustrative purposes only but not intended to limit the technical concept of the present disclosure. The scope of the technical concept of the present disclosure is not limited thereto. Therefore, it should be understood that the above-described exemplary embodiments are illustrative in all aspects and do not limit the present disclosure. The protective scope of the present disclosure should be construed based on the following claims, and all the technical concepts in the equivalent scope thereof should be construed as falling within the scope of the present disclosure.

[Explanation of Reference Numerals and Symbols]

> 100: Drug design device
> 110, 230: Storage unit
> 120, 210: Communication unit
> 130, 240: Processor
> 200: User mobile device
> 220: Display unit
> 410: Compound producing model
> 420: Agent network
> 430: Reward function
> 1000: Drug design system

[National R&D Project which supports this disclosure]

> [Project Identification Number] 1711106599
> [Project No.] 2018R1C1B6009531
> [Department] Ministry of Science and ICT
> [Project management (Professional) Institute] National Research Foundation of Korea
> [Research Project Name] Individual basic research (Ministry of Science and ICT)(R&D)
> [Research Task Name] Clinical-pathological and deep learning based analysis and research of diverse diseases in lung adenocarcinoma tumors
> [Contribution Ratio] 1/1
> [Project Performing Institution] Yeonsei University
> [Research Period] March 1, 2020 to February 28, 2021

**Claims**

1. A drug design method implemented by a processor and a communication unit, the method comprising:

   receiving a structure of a biological target, by means of the communication unit;
   performing, by means of the processor, reinforcement learning by using the binding affinity with the biological target, with respect to a compound producing model which has been pre-trained to output a molecular structure of a similar compound by inputting the molecular structure of the compound so as to obtain a reinforcement-learned compound producing model;
   producing a candidate compound for the biological target by means of the reinforcement-learned compound producing model; and
   providing the candidate compound.

2. The drug design method of claim 1, wherein the producing a candidate compound includes:

producing a plurality of candidate compounds using the reinforcement-learned compound producing model; determining a binding affinity for the biological target and each of the plurality of candidate compounds; and determining a candidate compound for the biological target, among the plurality of candidate compounds, based on the binding affinity for each thereof.

3. The drug design method of claim 2, wherein the determining a binding affinity includes:

determining a reward score for each thereof, using a reward function configured to evaluate a binding energy with a biological target, in a docking simulation environment, and
the determining a candidate compound includes:
determining a candidate compound for the biological target, among the plurality of candidate compounds, based on the reward score.

4. The drug design method of claim 3, wherein the determining a candidate compound includes:
determining a compound having a maximum reward score, among the plurality of candidate compounds, as a candidate compound for the biological target.

5. The drug design method of claim 3, wherein the reward function is configured to evaluate the binding affinity based on binding energy distribution of the previously received standard biological target and a standard compound.

6. The drug design method of claim 3, wherein the reward function is configured to determine the binding affinity score with a value between 0 and 1.

7. The drug design method of claim 3, wherein the reward function is configured to get a high reward score as the binding affinity with the biological target is lower, and
the determining a candidate compound further includes:
determining a candidate compound with a low binding affinity with the biological target, among the plurality of candidate compounds, based on the reward score.

8. The drug design method of claim 1, further comprising:

before the performing reinforcement learning,
receiving a molecular structure of a compound for training;

converting the molecular structure of the compound for training into a simplified molecular-input line-entry system (SMILES) code for training; and
training the compound producing model to output a similar SMILES code to the compound for training by inputting the SMILES code for training.

9. The drug design method of claim 8, wherein the producing a candidate compound includes:

producing a plurality of SMILES codes using the reinforcement-learned compound producing model;
determining a plurality of candidate compounds based on the plurality of SMILES codes;
determining a binding affinity for the biological target and each of the plurality of candidate compounds; and
determining a candidate compound for the biological target, among the plurality of candidate compounds, based on the binding affinity for each thereof.

10. The drug design method of claim 1, wherein the reinforced compound producing model includes an agent network, and
the performing reinforcement learning includes:
updating the agent network using a reward function configured to evaluate the binding affinity based on the binding energy with the biological target.

11. The drug design method of claim 1, wherein the biological target is at least one of virus protein, an active core site of coronavirus, and an active core site of severe acute respiratory syndrome coronavirus.

12. A drug design device, comprising:

a communication unit configured to receive a structure for a biological target; and
a processor configured to communicate with the communication unit, wherein the processor is configured to perform reinforcement learning by using the binding affinity with the biological target, with respect to a compound producing model which has been pre-trained to output a molecular structure of a similar compound by inputting the molecular structure of the compound so as to obtain a reinforcement-learned compound producing model, produce a candidate compound for the biological target by means of the reinforcement-learned compound producing model, and provide the candidate compound.

13. The drug design device of claim 12, wherein the processor is further configured to produce a plurality of candidate compounds using the reinforcement-learned compound producing model, determine a binding affinity for the biological target and each of the plurality of candidate compounds, and determine a candidate compound for the biological target, among the plurality of candidate compounds, based on the binding affinity for each thereof.

14. The drug design device of claim 13, wherein the processor is further configured to determine a reward score for each thereof, using a reward function configured to evaluate a binding energy with a biological target, in a docking simulation environment, and determine a candidate compound for the biological target, among the plurality of candidate compounds, based on the reward score.

15. The drug design device of claim 14, wherein the processor is further configured to determine a compound having a maximum reward score, among the plurality of candidate compounds, as a candidate compound for the biological target.

16. The drug design device of claim 14, wherein the reward function is configured to evaluate the binding affinity based on binding energy distribution of the previously received standard biological target and a standard compound.

17. The drug design device of claim 14, wherein the reward function is configured to determine the binding affinity score with a value between 0 and 1.

18. The drug design device of claim 14, wherein the reward function is configured to get a high reward score as the binding affinity with the biological target is lower, and
the processor is further configured to determine a candidate compound with a low binding affinity with the biological target, among the plurality of candidate compounds, based on the reward score.

19. The drug design device of claim 12, wherein the communication unit is further configured to receive a molecular structure of a compound for training, and
the processor is further configured to convert the molecular structure of the compound for training into a simplified molecular-input line-entry system (SMILES) code, and train the compound producing model to output a similar SMILES code to the compound for training by inputting the SMILES code for training.

20. The drug design device of claim 19, wherein the processor is further configured to produce a plurality of SMILES codes using the reinforcement-learned compound producing model, determine a plurality of candidate compounds based on the plurality of SMILES codes, determine a binding affinity for the biological target and each of the plurality of candidate compounds, and determine a candidate compound for the biological target, among the plurality of candidate compounds, based on the binding affinity for each thereof.

21. The drug design device of claim 12, wherein the reinforced compound producing model includes an agent network, and
the processor is further configured to update the agent network using a reward function configured to evaluate the binding affinity based on the binding energy with the biological target.

22. The drug design device of claim 12, wherein the biological target is at least one of virus protein, an active core site of coronavirus, and an active core site of severe acute respiratory syndrome coronavirus.

COVID-19 VIRUS SUPPRESSIVE CANDIDATE MATERIAL

CANDIDATE 1
REWARD SCORE: 0.8

CANDIDATE 2
REWARD SCORE: 0.74

CANDIDATE 3
REWARD SCORE: 0.73

1000

200

100

300

FIG. 1A

FIG. 1B

FIG. 1C

START

RECEIVE MOLECULAR STRUCTURE FOR BIOLOGICAL TARGET — S210

PERFORM REINFORCEMENT LEARNING USING BINDING AFFINITY WITH BIOLOGICAL TARGET, WITH RESPECT TO COMPOUND PRODUCING MODEL — S220

PRODUCE CANDIDATE COMPOUND FOR BIOLOGICAL TARGET USING REINFORCEMENT-LEARNED COMPOUND PRODUCING MODEL — S230

PROVIDE RESULT — S240

END

# FIG. 2A

410

PRE-TRAINED
COMPOUND
PRODUCING
MODEL

420

AGENT
NETWORK

GENERATE
SEQUENCE

DETERMINE
PRE-LIKELINESS

430

REWARD
FUNCTION

DETERMINE
AGENT-LIKELINESS

PRODUCE
SMILES

DETERMINE
BINDING AFFINITY

UPDATE
AGENT

FIG. 2B

(a)

(b)

FIG. 2C

```
┌─────────────────────────────────────────┐
│       PRODUCE PLURALITY OF CANDIDATE      │
│ COMPOUNDS USING REINFORCEMENT-LEARNED    │───S2100
│       COMPOUND PRODUCING MODEL            │
└─────────────────────────────────────────┘
                    │
                    ▼
┌─────────────────────────────────────────┐
│      DETERMINE REWARD SCORE FOR EACH OF   │
│  PLURALITY OF CANDIDATE COMPOUNDS USING   │
│       REWARD FUNCTION CONFIGURED TO       │───S2200
│        EVALUATE BINDING ENERGY WITH       │
│ BIOLOGICAL TARGET, IN DOCKING SIMULATION  │
│               ENVIRONMENT                 │
└─────────────────────────────────────────┘
                    │
                    ▼
┌─────────────────────────────────────────┐
│      DETERMINE CANDIDATE COMPOUND FOR     │
│    BIOLOGICAL TARGET, AMONG PLURALITY OF  │
│   CANDIDATE COMPOUNDS, BASED ON REWARD    │───S2300
│                 SCORE                     │
└─────────────────────────────────────────┘
```

# FIG. 2D

```
                    ┌─────────────┐
                    │    START    │
                    └──────┬──────┘
                           │
                           ▼
        ┌──────────────────────────────────────┐
        │   RECEIVE MOLECULAR STRUCTURE OF      │
        │     COMPOUND FOR TRAINING             │────~S310
        └──────────────────┬───────────────────┘
                           │
                           ▼
        ┌──────────────────────────────────────┐
        │   CONVERT MOLECULAR STRUCTURE OF      │
        │ COMPOUND FOR TRAINING INTO SMILES CODE│────~S320
        │            FOR TRAINING               │
        └──────────────────┬───────────────────┘
                           │
                           ▼
        ┌──────────────────────────────────────┐
        │  TRAIN COMPOUND PRODUCING MODEL TO    │
        │ OUTPUT SMILES CODE SIMILAR TO COMPOUND│
        │ FOR TRAINING, BY INPUTTING SMILES CODE│────~S330
        │            FOR TRAINING               │
        └──────────────────┬───────────────────┘
                           │
                           ▼
                    ┌─────────────┐
                    │     END     │
                    └─────────────┘
```

# FIG. 3

FIG. 4

FIG. 5A

Vina Score: 12.17(0.8) SMILES:
O_C(CC(CCC(-O)Nc1cc(C(F)(F)F)ccc1N1C[-O>cc2ccccc2C1-O)c1ccc(S(-O)(-O)NCc2ccccc2>cc1]c1cc(c2ccc3ccccc3c2)n[nH]1

Vina Score: 11.26(0.74) / SMILES:
O_C(Nc1cc([N+][-O][O-])ccc1OCc1cc([N+](-O[o-])>ccc1[N+](-O)[O-])Nc1ccccc1Oc1ccc(N+)(-O)F)cc1C(F)(F)F

Vina Score: -11.1(0.73) / SMILES:
O_C(NC[Cc1ccccc1)C(=O)NC(Cc1ccc([N+](=o)[O-])cc1)C(=O)Nc1ccc(C)]cc1)c1ccc(NC[=O]C2cc(-c3ccc4ccccc4c3)ccc2C1)cc1

FIG. 5B

| Chemble ID | Generic Name | Canonical SMILES |
|---|---|---|
| CHEMBL1957287 | CHEMBL1957287 | Fc1ccccc1-c1nc2ccn(Cc3ccc(-c4ccc(C(F)(F)F)cc4C(F)(F)F)nn3)cc-2n1 |
| CHEMBL2403318 | DELEOBUVIR | Cn1c(C2(NC(=O)c3ccc4c(C5CCCC5)c(-c5ncc(Br)cn5)n(C)c4c3)CCC2)nc2ccc(/C=C/C(=O)O)cc21 |
| CHEMBL3126842 | BECLABUVIR | COc1ccc2c(c1)[C@@H]1C[C@]1(C(=O)N1C3CCC1CN(C)C3)Cn1c-2c(C2CCCCC2)c2ccc(C(=O)NS(=O)(=O)N(C)C)cc21 |
| CHEMBL1092581 | NESBUVIR | CNC(=O)c1c(-c2ccc(F)cc2)oc2cc(N(CCO)S(C)(=O)=O)c(C3CC3)cc12 |
| CHEMBL490672 | FILIBUVIR | CCc1cc(CC[C@]2(C3CCCC3)CC(O)=C(Cc3nc4nc(C)cc(C)n4n3)C(=O)O2)cc(CC)n1 |
| CHEMBL3544985 | DASABUVIR SODIUM | COc1c(-c2ccc3cc(NS(C)(=O)=O)ccc3c2)cc(-n2ccc(=O)[n-]c2=O)cc1C(C)(C)C.O.[Na+] |
| CHEMBL3137325 | BECLABUVIR HYDROCHLORIDE | COc1ccc2c(c1)[C@@H]1C[C@]1(C(=O)N1C3CCC1CN(C)C3)Cn1c-2c(C2CCCCC2)c2ccc(C(=O)NS(=O)(=O)N(C)C)cc21.Cl |
| CHEMBL1076263 | SETROBUVIR | CS(=O)(=O)Nc1ccc2c(c1)S(=O)(=O)N=C(C1=C(O)[C@@H]3[C@H]4CC[C@H](C4)[C@@H]3N(Cc3ccc(F)cc3)C1=O)N2 |
| CHEMBL3137315 | DELEOBUVIR SODIUM | Cn1c(C2(NC(=O)c3ccc4c(C5CCCC5)c(-c5ncc(Br)cn5)n(C)c4c3)CCC2)nc2ccc(/C=C/C(=O)[O-])cc21.[Na+] |
| CHEMBL3039503 | LOMIBUVIR | CC(C)C#Cc1cc(N(C(=O)[C@H]2CC[C@H](C)CC2)[C@H]2CC[C@H](O)CC2)c(C(=O)O)s1 |
| CHEMBL3121539 | RADALBUVIR | CC1=CC[C@H](C(=O)N(c2cc(C#CC(C)(C)C)sc2C(=O)O)[C@H]2CC[C@](O)(CO[C@H]3CCOC3)CC2)CC1 |
| CHEMBL3137312 | DASABUVIR | COc1c(-c2ccc3cc(NS(C)(=O)=O)ccc3c2)cc(-n2ccc(=O)[nH]c2=O)cc1C(C)(C)C |
| CHEMBL1259059 | SOFOSBUVIR | CC(C)OC(=O)[C@H](C)N[P@]](=O)(OC[C@H]1O[C@@H](n2ccc(=O)[nH]c2=O)[C@](C)(F)[C@@H]1O)Oc1ccccc1 |
| CHEMBL3833371 | UPRIFOSBUVIR | CC(C)OC(=O)[C@@H](C)N[P@@](=O)(OC[C@H]1O[C@@H](n2ccc(=O)[nH]c2=O)[C@](C)(Cl)[C@@H]1O)Oc1ccccc1 |
| CHEMBL4129286 | ADAFOSBUVIR | CC(C)OC(=O)[C@H](C)N[P@](=O)(OC[C@@]1(F)O[C@@H](n2ccc(=O)[nH]c2=O)[C@](C)(O)[C@@H]1O)Oc1ccccc1 |
| CHEMBL1236524 | GALIDESIVIR | Nc1ncnc2c([C@@H]3N[C@H](CO)[C@@H](O)[C@H]3O)c[nH]c12 |
| CHEMBL4065616 | REMDESIVIR | CCC(CC)COC(=O)[C@H](C)N[P@](=O)(OC[C@H]1O[C@@](C#N)(c2ccc3c(N)ncnn23)[C@H](O)[C@@H]1O)Oc1ccccc1 |

FIG. 5C

| CHEMBLE ID | Generic Name | canonical smiles |
|---|---|---|
| CHEMBL114 | SAQUINAVIR | CC(C)(C)NC(=O)[C@@H]1C[C@@H]2CCCC[C@@H]2CN1C[C@@H](O)[C@@H](Cc1ccccc1)NC(=O)[C@H](CC(N)=O)NC(=O)c1ccc2ccccc2n1 |
| CHEMBL584 | NELFINAVIR | Cc1c(O)cccc1C(=O)N[C@@H](CSc1ccccc1)[C@H](O)CN1C[C@H]2CCCC[C@H]2C[C@@H]1C(=O)NC(C)(C)C |
| CHEMBL1664 | FOSAMPRENAVIR | CC(C)CN(C[C@@H](OP(=O)(O)O)[C@H](Cc1ccccc1)NC(=O)O[C@H]1CCOC1)S(=O)(=O)c1ccc(N)cc1 |
| CHEMBL1163 | ATAZANAVIR | InChI=1S/C38H52N6O7/c1-37(2,3)31(41-35(48)50-7)33(46)40-29(22-25-14-10-9-11-15-25)30(45)24-44(43-34(47)32(38(4,5)6)42-36(49)51-8)23-26-17-19-27(20-18-26)28-16-12-13-21-39-28/h9-21,29-32,45H,22-24H2,1-8H3,(H,40,46)(H,41,48)(H,42,49)(H,43,47)/t29-,30-,31+,32+/m0/s1 |
| CHEMBL1323 | DARUNAVIR | CC(C)CN(C[C@@H](O)[C@H](Cc1ccccc1)NC(=O)O[C@H]1CO[C@H]2OCC[C@@H]12)S(=O)(=O)c1ccc(N)cc1 |
| CHEMBL163 | RITONAVIR | CC(C)c1nc(CN(C)C(=O)N[C@@H](C(=O)N[C@@H](Cc2ccccc2)C[C@@H](O)[C@H](Cc2ccccc2)NC(=O)OCc2cncs2)C(C)C)cs1 |
| CHEMBL222559 | TIPRANAVIR | CCC[C@@]1(CCc2ccccc2)CC(O)=C([C@@H](CC)c2cccc(NS(=O)(=O)c3ccc(C(F)(F)F)cn3)c2)C(=O)O1 |
| CHEMBL115 | INDINAVIR | CC(C)(C)NC(=O)[C@@H]1CN(Cc2cccnc2)CCN1C[C@@H](O)C[C@@H](Cc1ccccc1)C(=O)N[C@H]1c2ccccc2C[C@@H]1O |
| CHEMBL116 | AMPRENAVIR | CC(C)CN(C[C@@H](O)[C@H](Cc1ccccc1)NC(=O)O[C@H]1CCOC1)S(=O)(=O)c1ccc(N)cc1 |
| CHEMBL2146090 | DANOPREVIR SODIUM | CC(C)(C)OC(=O)N[C@H]1CCCCC/C=C\[C@@H]2C[C@@]2(C(=O)[N-]S(=O)(=O)C2CC2)NC(=O)[C@@H]2C[C@@H](OC(=O)N3Cc4cccc(F)c4C3)CN2C1=O.[Na+] |
| CHEMBL3707372 | VOXILAPREVIR | CC[C@H]1[C@@H](C(=O)N[C@]2(C(=O)NS(=O)(=O)C3(C)CC3)C[C@H]2C(C)C)N2C[C@@H]1Oc1nc3cc(OC)ccc3nc1C(F)(F)CCCC[C@@H]1C[C@H]1OC(=O)N[C@@H](C(C)(C)C)C2=O |
| CHEMBL501849 | SIMEPREVIR | COc1ccc2c(O[C@@H]3C[C@H]4C(=O)N[C@]5(C(=O)NS(=O)(=O)C6CC6)C[C@H]5/C=C\CCCCN(C)C(=O)[C@@H]4C3)cc(-c3nc(C(C)C)cs3)nc2c1C |
| CHEMBL297884 | CILUPREVIR | COc1ccc2c(O[C@@H]3C[C@H]4C(=O)N[C@]5(C(=O)O)C[C@H]5/C=C\CCCC[C@H](NC(=O)OC5CCCC5)C(=O)N4C3)cc(-c3csc(NC(C)C)n3)nc2c1 |
| CHEMBL4297647 | FURAPREVIR | CC(C)Oc1ccc(-c2nc(O[C@@H]3C[C@H]4C(=O)N[C@]5(C(=O)NS(=O)(=O)C6CC6)C[C@H]5/C=C\CCCCC[C@H](NC(=O)OC5CCCC5)C(=O)N4C3)c3oc4ccccc4c3n2)cc1 |
| CHEMBL2105750 | SOVAPREVIR | C=C[C@@H]1C[C@]1(NC(=O)[C@@H]1C[C@@H](Oc2cc(-c3ccccc3)nc3cc(OC)ccc23)CN1C(=O)[C@@H](CC(=O)N1CCCC1)C(C)(C)C)C(=O)NS(=O)(=O)C1CC1 |
| CHEMBL3544915 | FALDAPREVIR SODIUM | C=C[C@@H]1C[C@]1(NC(=O)[C@@H]1C[C@@H](Oc2cc(-c3csc(NC(=O)C(C)C)n3)nc3c(Br)c(OC)ccc23)CN1C(=O)[C@@H](NC(=O)OC1CCCC1)C(C)(C)C)C(=O)[O-].[Na+] |
| CHEMBL599872 | VANIPREVIR | C=C[C@@H]1C[C@]1(NC(=O)[C@@H]1C[C@@H]2CN1C(=O)[C@H](C(C)(C)C)NC(=O)OCC(C)(C)CCCCc1cccc3c1CN(C3)C(=O)O2)C(=O)NS(=O)(=O)C1CC1 |
| CHEMBL2105735 | ASUNAPREVIR | C=C[C@@H]1C[C@]1(NC(=O)[C@@H]1C[C@@H](Oc2ncc(OC)c3ccccc23)CN1C(=O)[C@@H](NC(=O)OC(C)(C)C)C(C)(C)C)C(=O)NS(=O)(=O)C1CC1 |
| CHEMBL218394 | BOCEPREVIR | CC(C)(C)NC(=O)N[C@H](C(=O)N1C[C@H]2[C@@H]([C@H]1C(=O)NC(CC1CCC1)C(=O)C(N)=O)C2(C)C)C(C)(C)C |
| CHEMBL2063090 | GRAZOPREVIR | C=C[C@@H]1C[C@]1(NC(=O)[C@@H]1C[C@@H]2CN1C(=O)[C@H](C(C)(C)C)NC(=O)O[C@@H]1C[C@H]1CCCCc1nc3ccc(OC)cc3nc1O2)C(=O)NS(=O)(=O)C1CC1 |
| CHEMBL3391662 | PARITAPREVIR | Cc1cnc(C(=O)N[C@H]2CCCCC/C=C\[C@@H]3C[C@@]3(C(=O)NS(=O)(=O)C3CC3)NC(=O)[C@@H]3C[C@@H](Oc4nc5ccccc5c5ccccc45)CN3C2=O)cn1 |
| CHEMBL3040582 | DELDEPREVIR | COc1ccc2c(O[C@@H]3C[C@H]4C(=O)N[C@]5(C(=O)NS(=O)(=O)C6CC6)C[C@H]5/C=C\CCCCC[C@H](CC(=O)N5CCCC(F)(F)C5)C(=O)N4C3)cc(-c3nc(C(C)C)cs3)nc2c1C |
| CHEMBL2013174 | VEDROPREVIR | CC[C@@H]1C[C@]1(NC(=O)[C@@H]1C[C@@H](Oc2cc(-c3csc(NC(C)C)n3)nc3c(Cl)c(OCCN4CCOCC4)ccc23)CN1C(=O)[C@@H](NC(=O)O[C@@H]1C[C@@H]2C[C@@H]2C1)C(C)(C)C)C(=O)O |
| CHEMBL1255891 | NARLAPREVIR | CCCC[C@H](NC(=O)[C@@H]1[C@@H]2[C@@H](CN1C(=O)[C@@H](NC(=O)NC1(CS(=O)(=O)C(C)(C)C)CCCCC1)C(C)(C)C)C2(C)C)C(=O)C(=O)NC1CC1 |
| CHEMBL3039533 | GRAZOPREVIR | C=C[C@@H]1C[C@]1(NC(=O)[C@@H]1C[C@@H]2CN1C(=O)[C@H](C(C)(C)C)NC(=O)O[C@@H]1C[C@H]1CCCCc1nc3ccc(OC)cc3nc1O2)C(=O)NS(=O)(=O)C1CC1.O |
| CHEMBL3039499 | DELDEPREVIR SODIUM | COc1ccc2c(O[C@@H]3C[C@H]4C(=O)N[C@]5(C(=O)[N-]S(=O)(=O)C6CC6)C[C@H]5/C=C\CCCCC[C@H](CC(=O)N5CCCC(F)(F)C5)C(=O)N4C3)cc(-c3nc(C(C)C)cs3)nc2c1C.[Na+] |
| CHEMBL3137358 | SIMEPREVIR SODIUM | COc1ccc2c(O[C@@H]3C[C@H]4C(=O)N[C@]5(C(=O)[N-]S(=O)(=O)C6CC6)C[C@H]5/C=C\CCCCN(C)C(=O)[C@@H]4C3)cc(-c3nc(C(C)C)cs3)nc2c1C.[Na+] |
| CHEMBL3545363 | GLECAPREVIR | CC(C)(C)[C@@H]1NC(=O)[C@@H]2CCC[C@@H]2OC/C=C/C(F)(F)c2nc3ccccc3nc2O[C@@H]2C[C@@H](C(=O)N[C@]3(C(=O)NS(=O)(=O)C4(CC4)C[C@H]3C(F)F)N(C2)C1=O |
| CHEMBL258734 | DANOPREVIR | CC(C)(C)OC(=O)N[C@H]1CCCCC/C=C\[C@@H]2C[C@@]2(C(=O)NS(=O)(=O)C2CC2)NC(=O)[C@@H]2C[C@@H](OC(=O)N3Cc4cccc(F)c4C3)CN2C1=O |
| CHEMBL1241348 | FALDAPREVIR | C=C[C@@H]1C[C@]1(NC(=O)[C@@H]1C[C@@H](Oc2cc(-c3csc(NC(=O)C(C)C)n3)nc3c(Br)c(OC)ccc23)CN1C(=O)[C@@H](NC(=O)OC1CCCC1)C(C)(C)C)C(=O)O |
| CHEMBL282042 | SAQUINAVIR MESYLATE | CC(C)(C)NC(=O)[C@@H]1C[C@@H]2CCCC[C@@H]2CN1C[C@@H](O)[C@@H](Cc1ccccc1)NC(=O)[C@H](CC(N)=O)NC(=O)c1ccc2ccccc2n1.CS(=O)(=O)O |
| CHEMBL1201127 | DARUNAVIR ETHANOLATE | CC(C)CN(C[C@@H](O)[C@H](Cc1ccccc1)NC(=O)O[C@H]1CO[C@H]2OCC[C@@H]12)S(=O)(=O)c1ccc(N)cc1.CCO |
| CHEMBL3989607 | DROXINAVIR HYDROCHLORIDE | CNCC(=O)N[C@H](C(=O)N[C@@H](Cc1ccccc1)[C@H](O)CN(CCC(C)C)C(=O)NC(C)(C)C)C(C)C.Cl |
| CHEMBL206031 | BRECANAVIR | Cc1nc(COc2ccc(C[C@@H](NC(=O)O[C@@H]3CO[C@@H]4OCC[C@@H]34)[C@H](O)CN(CC(C)C)S(=O)(=O)c3ccc4c(c3)OCO4)cc2)cs1 |
| CHEMBL2103936 | TIPRANAVIR DISODIUM | CCC[C@@]1(CCc2ccccc2)CC([O-])=C([C@@H](CC)c2cccc([N-]S(=O)(=O)c3ccc(C(F)(F)F)cn3)c2)C(=O)O1.[Na+].[Na+] |
| CHEMBL1200678 | ATAZANAVIR SULFATE | COC(=O)N[C@H](C(=O)N[C@@H](Cc1ccccc1)[C@@H](O)CN(Cc1ccc(-c2ccccn2)cc1)NC(=O)[C@@H](NC(=O)OC)C(C)(C)C)C(C)(C)C.O=S(=O)(O)O |
| CHEMBL2104331 | LASINAVIR | COCCNC(=O)[C@@H](NC(=O)[C@H](Cc1ccc(OC)c(OC)c1OC)[C@H](O)[C@H](Cc1ccccc1)NC(=O)OC(C)(C)C)C(C)C |
| CHEMBL1205 | NELFINAVIR MESYLATE | CS(=O)(=O)O.Cc1c(O)cccc1C(=O)N[C@@H](CSc1ccccc1)[C@H](O)CN1C[C@H]2CCCC[C@H]2C[C@@H]1C(=O)NC(C)(C)C |
| CHEMBL13134 | PALINAVIR | CC(C)[C@H](NC(=O)c1ccc2ccccc2n1)C(=O)N[C@@H](Cc1ccccc1)[C@H](O)CN1C[C@H](OCc2ccncc2)C[C@@H]1C(=O)NC(C)(C)C |
| CHEMBL3990346 | DROXINAVIR | CNCC(=O)N[C@H](C(=O)N[C@@H](Cc1ccccc1)[C@H](O)CN(CCC(C)C)C(=O)NC(C)(C)C)C(C)C |
| CHEMBL223824 | MOZENAVIR | Nc1cccc(CN2C(=O)N(Cc3cccc(N)c3)[C@H](Cc3ccccc3)[C@H](O)[C@@H](O)[C@H](Cc2ccccc2)c1 |
| CHEMBL729 | LOPINAVIR | Cc1cccc(C)c1OCC(=O)N[C@@H](Cc1ccccc1)[C@@H](O)C[C@H](Cc1ccccc1)NC(=O)[C@H](C(C)C)N1CCCNC1=O |
| CHEMBL1200734 | FOSAMPRENAVIR CALCIUM | CC(C)CN(C[C@@H](OP(=O)([O-])[O-])[C@H](Cc1ccccc1)NC(=O)O[C@H]1CCOC1)S(=O)(=O)c1ccc(N)cc1.[Ca+2] |
| CHEMBL322241 | TELINAVIR | CC(C)CN(C[C@@H](O)[C@H](Cc1ccccc1)NC(=O)[C@@H](NC(N)=O)NC(=O)c1ccc2ccccc2n1)C(=O)NC(C)(C)C |
| CHEMBL1735 | INDINAVIR SULFATE | CC(C)(C)NC(=O)[C@@H]1CN(Cc2cccnc2)CCN1C[C@@H](O)C[C@@H](Cc1ccccc1)C(=O)N[C@H]1c2ccccc2C[C@@H]1O.O=S(=O)(O)O |
| CHEMBL2106751 | FOSAMPRENAVIR SODIUM | CC(C)CN(C[C@@H](OP(=O)([O-])[O-])[C@H](Cc1ccccc1)NC(=O)O[C@H]1CCOC1)S(=O)(=O)c1ccc(N)cc1.[Na+].[Na+] |

FIG. 5D

| – | prior | trained agent | difference |
|---|---|---|---|
| F | −8.47 | −7.62 | 0.85 |
| C | −6.46 | −5.71 | 0.75 |
| O | −6.60 | −6.01 | 0.59 |
| . | . | . | . |
| . | . | . | . |
| . | . | . | . |

FIG. 6A

| – | prior | trained agent | difference |
|---|---|---|---|
| . | . | . | . |
| . | . | . | . |
| . | . | . | . |
| Se | −19.05 | −20.65 | −1.59 |
| B | −12.48 | −14.35 | −1.87 |

FIG. 6B

# INTERNATIONAL SEARCH REPORT

| | |
|---|---|
| | International application No. |
| | **PCT/KR2022/002340** |

## A. CLASSIFICATION OF SUBJECT MATTER

**G16C 20/10**(2019.01)i; **G16C 20/30**(2019.01)i; **G16C 20/20**(2019.01)i; **G16C 20/70**(2019.01)i; **G06N 3/08**(2006.01)i; **G06N 20/00**(2019.01)i

According to International Patent Classification (IPC) or to both national classification and IPC

## B. FIELDS SEARCHED

Minimum documentation searched (classification system followed by classification symbols)

G16C 20/10(2019.01); A61K 47/48(2006.01); G16B 20/20(2019.01); G16B 25/10(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 약물설계 (Drug design), 생물학적 표적 (biological target), 결합 친화도 (binding affinity), 강화 학습 (reinforcement learning), 도킹 시뮬레이션 (docking simulation)

## C. DOCUMENTS CONSIDERED TO BE RELEVANT

| Category* | Citation of document, with indication, where appropriate, of the relevant passages | Relevant to claim No. |
|---|---|---|
| X<br>Y | JEON, W. et al. Autonomous molecule generation using reinforcement learning and docking to develop potential novel inhibitors. Scientific reports. 2020, vol. 10, thesis no. 22104, pp. 1-11.<br>    See Results and discussion; Conclusion; and methods. | 1-4,7,12-15,18<br>5-6,8-11,16-17,19-22 |
| Y | BOITREAUD, J. et al. OptiMol: optimization of binding affinities in chemical space for drug discovery. Journal of Chemical Information and Modeling. 2020, bioRxiv preprint doi: https://doi.org/10.1101/2020.05.23.112201. inner pp. 1-16.<br>    See sections 1~4. | 5,10,16,21 |
| Y | CHENTHAMARAKSHAN, V. et al. Cogmol: target-specific and selective drug design for covid-19 using deep generative models. Advances in Neural Information Processing Systems. 2020. arXiv:2004.01215v2. inner pp. 1-29.<br>    See sections 2, 4, 5 and 7. | 6,8-9,11,17,19-20,22 |
| A | KR 10-2020-0129367 A (KOREA UNIVERSITY RESEARCH AND BUSINESS FOUNDATION) 18 November 2020 (2020-11-18)<br>    See entire document. | 1-22 |

☑ Further documents are listed in the continuation of Box C.    ☑ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **30 May 2022** | **31 May 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/002340**

C. DOCUMENTS CONSIDERED TO BE RELEVANT

| Category* | Citation of document, with indication, where appropriate, of the relevant passages | Relevant to claim No. |
|---|---|---|
| A | WO 01-13958 A2 (BRENNER, S. et al.) 01 March 2001 (2001-03-01)<br>See entire document. | 1-22 |

Form PCT/ISA/210 (second sheet) (July 2019)

**INTERNATIONAL SEARCH REPORT**
Information on patent family members

International application No.

**PCT/KR2022/002340**

| Patent document cited in search report | | | Publication date (day/month/year) | Patent family member(s) | | | Publication date (day/month/year) |
|---|---|---|---|---|---|---|---|
| KR | 10-2020-0129367 | A | 18 November 2020 | KR | 10-2322884 | B1 | 05 November 2021 |
| WO | 01-13958 | A2 | 01 March 2001 | AU | 7082400 | A | 19 March 2001 |
| | | | | CA | 2382202 | A1 | 01 March 2001 |
| | | | | EP | 1212096 | A2 | 12 June 2002 |
| | | | | JP | 2003-507439 | A | 25 February 2003 |
| | | | | WO | 01-13958 | A3 | 31 January 2002 |

Form PCT/ISA/210 (patent family annex) (July 2019)